# EUROPEAN PATENT SPECIFICATION

(11) **EP 2 866 797 B1**
(45) Date of publication and mention of the grant of the patent: **29.04.2020**
(21) Application number: 12756353.4
(22) Date of filing: 06.07.2012
(51) Int. Cl.: A61K 31/165, A61K 9/00, A61K 9/70, A61K 31/5377, A61K 31/675

(54) **STABLE INJECTABLE PHARMACEUTICAL COMPOSITION OF NEUROKININ 1 RECEPTOR ANTAGONIST AND PROCESS FOR PREPARATION THEREOF**
STABILE INJIZIERBARE PHARMAZEUTISCHE ZUSAMMENSETZUNG EINES NEUROKININ-1-REZEPTORANTAGONISTEN UND VERFAHREN ZUR HERSTELLUNG DAVON
COMPOSITION PHARMACEUTIQUE INJECTABLE STABLE D'ANTAGONISTE DES RÉCEPTEURS À LA NEUROKININE 1 ET PROCÉDÉ DE PRÉPARATION ASSOCIÉ

(43) Date of publication of application: 06.05.2015
(73) Proprietor: Pharmathen S.A., 15351 Pallini Attikis (GR)
(72) Inventor: KARAVAS, Evangelos, GR-153 51 Pallini Attikis (GR); KOUTRIS, Efthimos, GR-153 51 Pallini Attikis (GR); BIKIARIS, Dimitrios, GR-54351 Tthessaloniki (GR); CHATIDOU, Sotiria, GR-153 51 Pallini Attikis (GR); DIAKIDOU, Amalia, GR-153 51 Pallini Attikis (GR); BARMPALEXIS, Panagiotis, GR-153 51 Pallini Attikis (GR); KONSTANTI, Louiza, GR-153 51 Pallini Attikis (GR); MINIOTI, Katerina, GR-153 51 Pallini Attikis (GR)
(86) International application number: PCT/EP2012/002854
(87) International publication number: WO 2014/005606

(56) References cited:
- WO-A1-2007/088483
- WO-A1-2011/006012
- WO-A1-2011/158053
- IAN OLVER ET AL: "Nanomedicines in the treatment of emesis during chemotherapy: focus on aprepitant", INTERNATIONAL JOURNAL OF NANOMEDICINE, DOVE MEDICAL PRESS LTD, AUCKLAND, NZ, vol. 2, no. 1, 1 May 2007 (2007-05-01), pages 13-18, XP009152304, ISSN: 1176-9114, DOI: 10.2147/IJN.S

## Description

### TECHNICAL FIELD OF THE INVENTION

The present invention relates to a stable controlled release injectable formulation containing a therapeutically effective quantity of a neurokinin 1 receptor antagonist, such as Aprepitant or Fosaprepitant or pharmaceutical acceptable salt, derivative or metabolite thereof and a process for manufacturing such composition.

### BACKGROUND OF THE INVENTION

Neurokinin 1 (NK1) antagonists are a novel class of medications that possess unique antidepressant, anxiolytic and antiemetic properties. The discovery of neurokinin 1 (NK1) receptor antagonists was a turning point in the prevention of nausea and vomiting associated with cancer chemotherapy.

Nausea and vomiting is an important adverse-effect of cancer chemotherapy treatment (CINV: Chemotherapy Induced Nausea and Vomiting syndrome). It is well documented that if not carefully treated with an antiemetic, more than 90% of cancer chemotherapy treated patients experience in some extent CINV symptoms. The syndrome may have a significant impact on the treatment and the quality of life of patients as it may be responsible for delaying or even in some cases canceling scheduled chemotherapies. Generally, CINV syndrome can be categorized as a) acute, b) delayed and c) anticipatory. In acute CINV, nausea and vomiting appear within the first 24 hours after chemotherapy, while in the case of delayed CINV, symptoms can last for several days. Anticipatory CINV is a conditional response that occurs in patients who had poorly controlled CINV symptoms during a previous course of chemotherapy.

Until recently the prevention and treatment of CINV symptoms involved the use of corticosteroids, dopamine D2 antagonists and serotonin 5-HT3 receptor antagonists. Therapies consisting of serotonin 5-HT3 receptors (such as Kytril® (granisetron), Zofran® (ondansetron), Anzemet® (dolesetron), Aloxi® (palonosetron) and Navoban® (Tropisetron)), are all equally effective in the prevention of acute CINV symptoms only. Aloxi® (palonosetron), due to its longer half life, is the only serotonin 5-HT3 receptor antagonist currently approved for the prevention of both acute and delayed CINV. Usually, a combination of Aloxi® and a corticosteroid, administrated prior to chemotherapy, followed by administration of one or both agents for several days is used for the prevention of delayed CINV. However, unpleasant adverse-effects due to the multiple dosing of 5-HT3 receptor antagonist during the treatment of delayed CINV symptoms, such as headache and constipation, led to the addition of neurokinin 1 receptor antagonists, such as Aprepitant or Fosaprepitant, in the treatment regimen.

Aprepitant is an active pharmaceutical ingredient categorized as a highly-selective antagonist of neurokinin 1 receptors with little or no affinity for serotonin, dopamine or corticosteroid receptors. Aprepitant is administrated orally at a dose of 125 mg once daily on day one and 80 mg once daily on days two and three. Intravenously (IV) Aprepitant is administrated in the form of Fosaprepitant Dimeglumine, a water-soluble phosphorylated prodrug of Aprepitant, which is rapidly converted to Aprepitant in vivo. The molecular formula of Fosaprepitant Dimeglumine is C₂₃H₂₂F₇N₄O₆P•2C₇H₁₇NO, corresponding to a molecular weight of 1004.83. It is a hygroscopic, freely soluble in water, white to off-white amorphous powder intended for intravenous infusion.

US 2007/265329 A1 discloses a subcutaneous pharmaceutical injectable composition comprising a semi-solid delivery vehicle and a pharmaceutically acceptable liquid vehicle, for the sustained release of a 5-HT3 receptor antagonist in the treatment of CINV.

EP 0 748 320 B1 discloses the use of neurokinin 1 receptor antagonist for the treatment of emesis.

US 5538982 B1 discloses a combination of a neurokinin 1 receptor antagonist and a 5-HT3 receptor antagonist for the treatment of emesis.

WO 2011/158053 A1 discloses nanostructured Aprepitant and its pharmaceutical composition with increased solubility/dissolution rate and bioequivalence as compared to reference active compound and to the marketed product.

Although each of the patents above represents an attempt to overcome the problems associated with existing treatment regimens of chemotherapy-induced nausea and vomiting (CINV), there still exists a need for a long-acting injectable formulation with reduced side effects.

### SUMMARY OF THE INVENTION

It is, therefore, an object of the present invention to provide a controlled release sterile injectable formulation containing a neurokinin 1 receptor antagonist, and in particular Aprepitant or Fosaprepitant or a pharmaceutical acceptable salt, derivative or metabolite thereof, as an active ingredient, which overcomes the deficiencies of the prior art and provides a uniform and constant rate of release over an extended period of time.

It is another object of the present invention to provide a stable parenteral pharmaceutical formulation comprising Aprepitant or Fosaprepitant, as an active ingredient, which is free from pyrogens and fever-producing substances.

Another aspect of the present invention is to provide a parenteral formulation comprising Aprepitant or Fosaprepitant which is isotonic with the biological fluids into which it is injected (intramuscularly and/or subcutaneously) and non-irritating.

Further object of the present invention is to provide an injectable controlled release formulation comprising Aprepitant or Fosaprepitant or a pharmaceutical acceptable salt, derivative or metabolite thereof, as an active ingredient, which shows good syringability, injectability, no clogging or blockage of the syringe needles, good drainage, sterility and re-suspendibility (in case of suspensions).

A major object of the present invention is to provide a controlled release injectable formulation of Aprepitant or Fosaprepitant, which is able to replace the existing CINV treatment regimens, and hence annihilate the side-effects induced by the increased amounts of corticosteroids administrated in the "single dosing" regimen, while reducing patience non-compliance to medication and discomfort, adverse effects related to the frequent oral dosing of Aprepitant capsules according to the "3-Day dosing" regimen.

The present invention aims at developing a pharmaceutical composition for intramuscular or subcutaneous administration, in a single or multiple injection sites, comprising Aprepitant or Fosaprepitant or a pharmaceutical acceptable salt, derivative or metabolite thereof, as an active ingredient, which is able to prevent, reduce or alleviate acute, delayed and anticipatory CINV symptoms from day 1 (beginning of the chemotherapy treatment) and up to several days.

The invention further includes sterile injectable controlled release formulations comprising Aprepitant or Fosaprepitant, as an active ingredient, in the form of: 1) ready to use solutions 2) ready to use suspensions 3) other specific injectable pharmaceutical formulations such as sol-gel formulations.

A further approach of the present invention is to provide a fast, simple and cost-effective process for the preparation of a stable injectable pharmaceutical formulation comprising Aprepitant or Fosaprepitant or a pharmaceutical acceptable salt, derivative or metabolite thereof.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient (e.g. Aprepitant or Fosaprepitant) is considered to be "stable" if said ingredient degrades less or more slowly than it does on its own and/or in known pharmaceutical compositions.

As already mentioned the main object of the present invention is to provide a controlled release injectable formulation of Aprepitant or Fosaprepitant or a pharmaceutical acceptable salt, derivative or metabolite thereof, which minimizes the side effects associated with the existing treatment regiments of chemotherapy-induced nausea and vomiting (CINV).

The current dose regimen for the treatment of CINV symptoms depends on the Fosaprepitant injection dose. For a "single dose" regimen, 245.3 mg Fosaprepitant Dimeglumine injection (equivalent to 150 mg of Fosaprepitant free acid) is administrated intravenously (IV) on Day 1, as infusion over 20 to 30 minutes, initiated 30 minutes prior to chemotherapy. The therapeutic regimen in this case, which also includes a 5-HT₃ antagonist and a corticosteroid, is given in Table 1.

**Table 1: Single Dose Regimen of Aprepitant/Fosaprepitant for prevention of CINV**

| | Day 1 | Day 2 | Day 3 | Day 4 |
|---|---|---|---|---|
| Aprepitant/Fosaprepitant | 150 mg IV | none | none | none |
| Corticosteroid | 12 mg orally | 8 mg orally | 8 mg orally twice a day | 8 mg orally twice a day |
| 5-HT3 antagonist | 32 mg IV | none | none | none |

For a "3-Day dosing" regimen, 188 mg Fosaprepitant Dimeglumine injection (equivalent to 115 mg of Fosaprepitant free acid) is administrated intravenously (IV) on Day 1 as infusion over 15 minutes, initiated 30 minutes prior to chemotherapy. Capsules of 80 mg Aprepitant are to be administrated on Days 2 and 3. The therapeutic regimen in this case also includes a 5-HT3 antagonist and a corticosteroid (Table 2).

**Table 2: 3-Day Dosing Regimen of Aprepitant/Fosaprepitant for prevention of CINV**

| | Day 1 | Day 2 | Day 3 | Day 4 |
|---|---|---|---|---|
| Aprepitant/Fosaprepitant | 115 mg IV | 80 mg orally | 80 mg orally | none |
| Corticosteroid | 12 mg orally | 8 mg orally | 8 mg orally once a day | 8 mg orally once a day |
| 5-HT3 antagonist | 32 mg IV | none | none | none |

From the above dose regimens it is apparent that when 245.3 mg of Fosaprepitant Dimeglumine are injected intravenously in Day 1 ("single dosing" regimen) no extra orally administrated Aprepitant is needed. However, in this case, an extra amount of corticosteroids is administrated. Keeping in mind that corticosteroids are drugs that cause serious side-effects, it is desirable to establish a treatment regimen for CINV symptoms able to reduce the total amounts of corticosteroids administrated in the "single dosing" regimen without increasing patience non-compliance and discomfort (problem associated with multiple oral dose administration of Aprepitant capsules in "3-Day dosing" regimen).

According to the present invention, the controlled release injectable, intramuscular (IM) or subcutaneous (SC), formulation of Aprepitant or Fosaprepitant Dimeglumine replaces both "single dosing" and "3-Day dosing" regimens. Said formulation not only eliminates problems related to the "3-Day dosing" regimen, such as patients non-compliance to medication and discomfort, but also reduces problems associated with the "single dosing" (increased cumulative amount of corticosteroids).

Controlled release parenteral drug products according to the present invention may be available as solutions (aqueous and non-aqueous), emulsions (aqueous and non-aqueous), powder for suspension, coarse or colloidal liquid suspensions.

Controlled release injectable IM and/or SC formulations of Aprepitant or Fosaprepitant or pharmaceutical acceptable salt, derivative or metabolite thereof, as the active ingredient, can be produced when a viscosity increasing agent (or a mixture of agents) is added in an aqueous solution. The viscosity increasing agent is able to delay the active ingredients' dissolution profile for several days. Specifically the viscosity increasing agents can be one of sodium carboxymethylcellulose (SCMC) or hydroxyl propyl methyl cellulose (HPMC).

The pharmaceutically acceptable aqueous controlled release sterile injectable can have concentration of from 0.001 wt. % to 90 wt.%, preferably from 0.1 wt.% to 30 wt.%. and may comprise one or more solubilizing or wetting agents, one or more viscosity increasing agents, one or more buffering agents, one or more pH adjusting agents, and one or more tonicity adjusting agents.

The solubilizing or wetting agents may have a concentration varying from 0.01wt.% to 50 wt.%, based on the total weight of the sterile controlled release injectable formulation. Examples of suitable solubilizing agents include one or mixture of the following: diethylene glycol monostearate, diethylene glycol monolaurate, glyceryl monostearate, polyoxyethylene sorbitol beeswax, polyethylene lauryl ether, polyoxyethylene leuryl ether, polyoxyethylene monostearate, polyoxyethylene alkyl phenol, polyethylene sorbitan monooleate, polyethylene sorbitan monolaurate, polyoxyethylene lauryl ether, potassium oleate, sorbitan tristearate, sorbitan monolaurate, sorbitan monooleate, sodium lauryl sulfate, sodium oleate, triethanolamine oleate, etc. Poloxamer and Lutrol® F108 are particularly preferred as solubilizing or wetting agents.

The viscosity increasing agents may have a concentration varying from 0.01 mg-mL⁻¹ to 350 mg-mL⁻¹, preferably from 1 mg-mL⁻¹ to 150 mg-mL⁻¹. Examples of suitable viscosity increasing agents include one or mixture of the following: aluminum monostearate, carboxy methylcellulose, desoxycholate sodium, gelatin, glycerol, hydroxyethyl cellulose, hydroxypropylmethylcellulose, lecithin, polyoxyethylene alkyl ethers, poloxamer, polyoxyethylated fatty acid, polysorbate, polyethylene glycol, polyvinyl pyrrolidone, sodium carboxymethylcellulose etc. Sodium carboxymethylcellulose or hydroxypropylmethylcellulose are particularly preferred as viscosity increasing agents.

A buffer may be optionally employed, in a specific amount as to adjust the pH value from about 6 to about 8, preferably about 7. To achieve such pH values, usually the buffer is present at a concentration level varying from 0.02 wt.% to 2 wt.%, preferably from 0.03 wt.% to 1 wt.%, more preferably from 0.05 wt.% to 0.5 wt.%, based on the total weight of the sterile controlled release injectable formulation. Examples of suitable buffers include: sodium phosphate and potassium phosphate. Sodium phosphate is particularly preferred as buffering agent.

The aqueous ready to use controlled release injectable solution of the present invention may optionally include one or more pH adjusting agents. The pH adjusting agents may be either an acid or a base. Examples of pH adjusting agents include one or mixture of the following: acetic acid, calcium carbonate, hydrochloric acid, magnesium oxide, magnesium hydroxide, potassium hydroxide, sodium hydroxide etc. Sodium hydroxide and hydrochloric acid are particularly preferred as pH adjusting agents.

Additionally, one or more tonicity adjusting agent may be optionally added. Examples of suitable tonicity adjusting agents include magnesium sulfate, maltose, mannitol, polyethylene glycol, polylactic acid, polysorbate, potassium chloride, povidone, sodium chloride, sodium cholesteryl sulfate, sodium succinate, sodium sulfate, sorbitol, sucrose, trehalose etc. Sodium chloride is particularly preferred, when necessary, as tonicity adjusting agent.

The controlled release injectable IM and/or SC formulations in the form of an aqueous solution is a sterile, pyrogen free, stable, isotonic and non-irritating ready to use solution.

Controlled release injectable IM and/or SC formulations of Aprepitant or Fosaprepitant or pharmaceutical acceptable salt, derivative or metabolite thereof, as the active ingredient, can be also produced in the form of a suspension, either colloidal or coarse. Such formulations are heterogeneous systems consisting of a solid phase at a concentration varying from 0.5 wt.% to 40 wt.% based on the total weight of the formulation in an non-aqueous liquid phase. Colloidal suspensions are suspensions in which the particle size is small enough, less than 1µm, where the suspended phase does not settle under the force of gravity, while coarse suspensions typically contain dispersed solid particle with a size greater than 1µm.

The solid phase may be in the form of a powder physical mixture, granule, solid dispersion, self-emulsifying system, micro-nano spheres, micro-nano capsules, pellets or lyophilized cake.

The liquid vehicle of the controlled release injectable suspension is a non-aqueous liquid, present at a concentration varying from about 0.01 wt.% to about 99 wt.%.

The proposed ready to use injectable suspension may be formulated as: controlled flocculated system, structured vehicle, or combinations thereof. The choice depends on whether the particles in the suspension are to remain flocculated or deflocculated. In the controlled flocculation approach, flocculating agents are added in order to form loosely bound aggregates of floes in a controlled manner that settles rapidly but re-disperses easily upon agitation. Typical flocculating agents include a combination of electrolytes (such as potassium/sodium chloride, citrate and acetate etc.), surfactants (such as lecithin, polysorbates, pluronic, sorbitan trioleate etc.) and hydrophilic colloids (such as sodium carboxy methyl cellulose, acacia, gelatin, methylcellulose, polyvinylpyrrolidone etc.) Injectable suspensions in the form of structure vehicles use suspending or thickening agents in order to keep the dispersed particles in a deflocculated state.

The advantages of a controlled release injectable suspension include a) the ability of therapeutically using active ingredients that are insoluble in conventional solvents, b) increased resistance to hydrolysis and oxidation, and c) elimination of hepatic first-pass effect.

The proposed injectable suspensions show good drainage (an attribute characterized as the ability of the suspension to break cleanly away from the inner walls of the primary container-closure). Silicone coating of containers, vials and plugs with dimethicone may be used when necessary to improve drainage and reduce potential particle agglomeration on the container's surfaces.

According to the present invention, sesame and/or castor oils are used as non-aqueous water-immiscible liquid vehicles for the preparation of a pharmaceutically acceptable sterile controlled release injectable ready to use suspension at a concentration varying from 0.01 wt.% to 99.9 wt.%, preferably from 50 wt.% to 95 wt.%.

The pharmaceutically acceptable non-aqueous controlled release sterile injectable ready to use suspension can have an active ingredient concentration of from 0.001 wt. % to 50 wt.%, preferably from 0.1 wt.% to 30 wt.%. and may also comprise one or more solubilizing or wetting agents, one or more flocculating or suspending agents, one or more antimicrobial preservatives, one or more antioxidants, one or more buffering agents, one or more pH adjusting agents, one or more tonicity adjusting agents, and one or more chelating agents.

The solubilizing or wetting agents may be present at a concentration varying from 0.01 wt.% to 50 wt.%,. Examples of suitable solubilizing agents include diethylene glycol monostearate, diethylene glycol monolaurate, glyceryl monostearate, polyoxyethylene sorbitol beeswax, polyethylene lauryl ether, polyoxyethylene leuryl ether, polyoxyethylene monostearate, polyoxyethylene alkyl phenol, polyethylene sorbitan monooleate, polyethylene sorbitan monolaurate, polyoxyethylene lauryl ether, potassium oleate, sorbitan tristearate, sorbitan monolaurate, sorbitan monooleate, sodium lauryl sulfate, sodium oleate, triethanolamine oleate, etc Poloxamer and Lutrol® F108 are particularly preferred as solubilizing or wetting agents.

The flocculating or suspending agents may be present at a concentration varying from 0.01 mg-mL⁻¹ to 350 mg·mL⁻¹, preferably from 1 mg-mL⁻¹ to 150 mg·mL⁻¹. Sodium carboxymethylcellulose and hydroxypropylmethylcellulose are particularly preferred as flocculating or suspending agents.

Antimicrobial preservatives may also be present in the sterile controlled release injectable suspension. Examples of antimicrobial preservatives include benzalkonium chloride, benzyl alcohol, chlorbutanol, m-cresol, myristyl gamma-picolinium chloride, methyl and propyl paraben, phenol, 2-phenoxyethanol, phenyl mercuric nitrate, thimerosal etc. Methyl and propyl paraben are particularly preferred as antimicrobial preservatives.

Examples of antioxidants that may also be present include acetone sodium bisulfate, ascorbate, α-tocopherol, bisulfate sodium, butylated hydroxy anisole, butylated hydroxy toluene, cystein, cysteinate HCl, dithionite sodium, gentisic acid, gentisic acid athanolamine, glutamate monosodium, formaldehyde sulfoxylate sodium, metabisulfite potassium, metabisulfite sodium, monothioglycerol, propyl gallate, sulfite sodium, tocopherol alpha, thioglycolate sodium etc. Butylated hydroxyl anisole and/or bisulfite are particularly preferred as antioxidants.

For the preparation of the non-aqueous ready to use controlled release injectable suspension, a buffer is optionally employed in a specific amount as to adjust the pH value from about 6 to about 8, preferably about 7. To achieve such pH values, usually the buffer is present at a concentration level varying from 0.02 wt.% to 2 wt.%, preferably from 0.03 wt.% to 1 wt.%, more preferably from 0.05 wt.% to 0.5 wt.%, based on the total weight of the sterile controlled release injectable formulation. Examples of suitable buffers include: sodium phosphate, potassium phosphate, sodium hydroxide, succinate sodium, succinate disodium, sulfuric acid, tertrate sodium, tertrate acid, tromathamine etc. Sodium phosphate is particularly preferred as buffering agent.

One or more pH adjusting agents in order to adjust the pH of the formulation from about 6 to about 8, preferably about 7, may be present as well. The pH adjusting agents may be either an acid or a base. Examples of pH adjusting agents suitable for use in the present invention include acetic acid, calcium carbonate, hydrochloric acid, magnesium oxide, magnesium hydroxide, potassium hydroxide, sodium hydroxide etc. Sodium hydroxide and hydrochloric acid are particularly preferred as pH adjusting agents.

For the preparation of the controlled release injectable suspension, one or more tonicity adjusting agent is optionally used. Examples of suitable tonicity adjusting agents include magnesium sulfate, maltose, mannitol, polyethylene glycol, polylactic acid, polysorbate, potassium chloride, povidone, sodium chloride, sodium cholesteryl sulfate, sodium succinate, sodium sulfate, sorbitol, sucrose, trehalose etc. Sodium chloride is particularly preferred as tonicity adjusting agent.

The proposed controlled release injectable suspension of the present invention may optionally include one or more chelating agents. Examples of chelating agents suitable for use in the present invention include calcium disodium ethylenediaminetraacetic acid (EDTA), disodium EDTA, sodium EDTA and diethylenetriaminepentaacetic acid (DTPA) etc. Citric acid, tartaric acid and some amino acids can also act as chelating agents. Disodium EDTA is particularly preferred as chelating agent.

Controlled release injectable IM and/or SC formulations of Aprepitant or Fosaprepitant or pharmaceutical acceptable salt, derivative or metabolite thereof, as the active ingredient, can be also produced in the form of a liquid able to transform in situ into a solid or gel implant (sol-gel transformation).

Said sol-gel formulation includes a polymer which is substantially or completely insoluble in an aqueous medium or body fluid. This thermoplastic polymer may be a homopolymer, a copolymer or a terpolymer of repeating monomeric units linked by such groups as ester, anhydride, carbonate, amide, urethane, urea, ether, esteramide, acetal, ketal, orthocarbonate and any other organic functional group that can be hydrolyzed by enzymatic or hydrolytic reaction (i.e. biodegradable by a hydrolytic reaction). The polymer may be a polyester or copolymer of hydrolytic biocompatible PEG with biodegradable polyesters, such as polylactide (PLA), polyglycolide (PGA), poly(ε-caprolactone) (PCL), poly[(R)-3-hydroxybutyrate] (PHB), polyphosphazenes or block copolymers of ethylene oxide and propylene oxide polyphosphazenes, polypeptides, polysaccharides, such as chitosan, poly(trimethylene carbonate) (PTMC), acidic sulfamethazine oligomers (OSMs), basic poly(β-amino ester) (PAE), poly(amino urethane) (PAU) or poly(aminoamine) (PAA).

In particular the polymer may be polyester (or a co-polymer of it) composed of units of about one or more hydroxyl-carboxylic acid residues, or diol and dicarboxylic acid residues, wherein the distribution of the different residues may be random, block, paired or sequential.

The polyester may be a combination of about one or more diols and about one or more dicaroxylic acids. The hydroxyl carboxylic acid or acids may also be in the form of a dimer.

In one aspect, the biodegradable polymer is a copolymer of an aliphatic polyester. Aliphatic polyesters are synthetic homopolymers or copolymers of lactic acid, glycolic acid, lactide, glycolide and e-hydroxycaproic acid. In particular the polyesters may include a polylactide, a polyglycolide, a polycaprolactone, a copolymer thereof, a tetropolymer thereof, or any combination thereof, optionally incorporating a third mono-alcohol or polyol component having an acid, ester or alkyl ester terminated group.

The biodegradable polymer may also be a di-block or a tri-block copolymer of PEG with an aliphatic-polyester. Examples of di-block copolymers are: PEG-PLLA (poly(L-lactide)), PEG-PLA (poly(DL-lactide)), PEG-PLGA (poly(L-lactide-co-glycolide), or poly(DL-lactide-co-glycolide)), PEG-PCL (poly(ε-caprolactone)), PEG-PLCL (poly(L-lactide-co-ε-caprolactone)), PEG-DLPLCL (poly(DL-lactide-co-ε-caprolactone)). Examples of tri-block copolymers are: 1) ABA-type copolymers, such as PEG-PLLA-PEG, PEG-PLA-PEG, PEG-PLGA-PEG, PEG-PCL-PEG, PEG-DLPLCL-PEG and 2) BAB-type copolymers, such as PLLA-PEG-PLLA, PLA-PEG-PLA, PLGA-PEG-PLGA, PCL-PEG-PCL, DLPLCL-PEG-DLPLCL. PLGA and PLCL copolymers may contain L-lactide from 0.1 wt.% to 100 wt.%, DL-lactide from 0.1 wt.% to 100 wt.%, glycolide from 0 wt.% to 99.9 wt.% and caprolactone from 0 wt.% to 100 wt.%.

Self-assembling block copolymers, such as the above mentioned, have the ability to form temperature dependent micellar aggregates and after a further temperature increase, they form gels due to micelles aggregation or packing. Therefore, it is possible to prepare a pharmaceutical formulation which is in the form of a solution at room temperature and when injected to a selected tissue, to form in-situ a gel depot able to provide a delayed release for the active ingredient.

The pharmaceutically acceptable controlled release injectable sol-gel formulation, according to the present invention, varies in a concentration of from 0.0001 wt. % to 70 wt% and consists of: one or more biodegradable polymers or copolymers, one or more solubilizing or wetting agents, optionally one or more buffering agents, optionally one or more pH adjusting agents, and optionally one or more tonicity adjusting agents.

The biodegradable polymers, or copolymers, can be present in any suitable amount varying from 0.01 wt.% to 99 wt.%, with a molecular weight form 1,000 Da up to over 100,000 Da. Example of suitable temperature-sensitive or pH/temperature-sensitive biodegradable copolymer are PEG-PLLA that is used as a di-block biodegradable copolymer.

The solubilizing or wetting agents may be present at a concentration varying from 0.01 wt% to 50 wt% based on the total weight of the sterile controlled release injectable formulation. Examples of suitable solubilizing agents include diethylene glycol monostearate, diethylene glycol monolaurate, glyceryl monostearate, polyoxyethylene sorbitol beeswax, polyethylene lauryl ether, polyoxyethylene leuryl ether, polyoxyethylene monostearate, polyoxyethylene alkyl phenol, polyethylene sorbitan monooleate, polyethylene sorbitan monolaurate, polyoxyethylene lauryl ether, potassium oleate, sorbitan tristearate, sorbitan monolaurate, sorbitan monooleate, sodium lauryl sulfate, sodium oleate, triethanolamine oleate, etc Poloxamer and Lutrol F108 are particularly preferred as solubilizing or wetting agents.

For the preparation of the sol-gel controlled release injectable formulation, a buffer may also be included in a specific amount as to adjust the pH value from about 6 to about 8, preferably about 7. To achieve such pH values, usually the buffer is present at a concentration level varying from 0.02 wt.% to 2 wt.%, preferably from 0.03 wt.% to 1 wt%, more preferably from 0.05 wt.% to 0.5 wt.%, based on the total weight of the sterile controlled release injectable formulation. Examples of suitable buffers for use in the present invention include. sodium phosphate, potassium phosphate, sodium hydroxide, succinate sodium, succinate disodium, sulfuric acid, tertrate sodium, tertrate acid, tromathamine etc. Sodium phosphate is particularly preferred as buffering agent.

The sol-gel controlled release injectable formulation of the present invention may optionally include one or more pH adjusting agents in order to adjust the pH of the formulation from about 6 to about 8, preferably about 7. The pH adjusting agents may be either an acid or a base. Examples of pH adjusting agents suitable for use in the present invention include acetic acid, calcium carbonate, hydrochloric acid, magnesium oxide, magnesium hydroxide, potassium hydroxide, sodium hydroxide etc. Sodium hydroxide and hydrochloric acid are particularly preferred as pH adjusting agents.

For the preparation of the sol-gel controlled release injectable formulation, one or more tonicity adjusting agent is optionally added. Examples of suitable tonicity adjusting agents include magnesium sulfate, maltose, mannitol, polyethylene glycol, polylactic acid, polysorbate, potassium chloride, povidone, sodium chloride, sodium cholesteryl sulfate, sodium succinate, sodium sulfate, sorbitol, sucrose, trehalose etc. Sodium chloride is particularly preferred as tonicity adjusting agent.

According to an object of the present invention a process for preparing a controlled release pharmaceutical composition for intramuscular or subcutaneous administration, comprising Aprepitant or Fosaprepitant or a pharmaceutical acceptable salt, derivative or metabolite thereof, as an active ingredient, is also provided.

Said process should be fast, simple and cost-effective. The composition of the present invention comprises the active pharmaceutical ingredient, a vehicle, other pharmaceutically acceptable excipients and pH adjusting agent(s). The order of mixing the constituents of the composition is interchangeable. According to the present invention the process may comprise the following steps:
- Weighted amounts of the active ingredient and liquid vehicle (aqueous or non-aqueous) are mixed into a glass vial or a beaker and stirred for approximately thirty minutes until a clear solution is achieved;
- Any optional pharmaceutically acceptable excipient such as a viscosity enhancing agent or biodegradable polymer is added and stirred for another thirty minutes;
- Finally, the pH of the final solution is adjusted to approximately seven.

Alternatively, the process comprises the following steps:
- Weighted amounts of any pharmaceutically acceptable excipient such as a viscosity enhancing agent or biodegradable polymer and liquid vehicle (aqueous or non-aqueous) are mixed into a glass vial, or a beaker and stirred for approximately thirty minutes until a clear solution is achieved;
- The pharmaceutically active ingredient is added and stirred for another thirty minutes;
- Finally, the pH of the final solution is adjusted to approximately seven.

Finally, the process may comprise the following steps:
- Two distinct solutions are prepared. A first solution is prepared by mixing weighted amounts of any pharmaceutically acceptable excipient such as a viscosity enhancing agent or biodegradable polymer and liquid vehicle (aqueous or non-aqueous) into a glass vial, or a beaker and stirred for approximately thirty minutes;
- A second solution is prepared by mixing weighted amounts of the active ingredient and liquid vehicle (aqueous or non-aqueous) into a glass vial, or a beaker and stirring for approximately thirty minutes;
- Finally, the two solutions are then mixed together at appropriate amounts. The pH of the final solution is adjusted to approximately seven.

The following examples illustrate preferred embodiments in accordance with the present invention without intending to limit the scope or spirit of the invention.

### EXAMPLES

### Example 1

Aqueous ready to use solutions according to the present invention are illustrated in Table 3:

**Table 3: Controlled release aqueous ready to use injectable solutions**

| | Reference (g/L) | Formulation A (g/L) | Formulation B (g/L) |
|---|---|---|---|
| Fosaprepitant Dimeglumine | 142.7 | 142.7 | 142.7 |
| Hydroxypropylmethylcellulose K100 | - | 6.7 | - |
| Sodium carboxymethylcellulose | - | - | 6.7 |
| Water for injection | q.s to 1L | q.s to 1L | q.s to 1L |

Sodium Phosphate and NaOH or HCl were used at appropriate amounts in order to adjust pH at 7.

The prepared formulations of Table 3 showed good syringability and injectability with no clogging or blockage of the syringe needles.

The controlled release ready to use injectable solutions of Fosaprepitant Dimeglumine of example 1, was prepared by the following process:
- Weighted amounts of Fosaprepitant Dimeglumine and water were mixed into a glass vial, or a beaker and stirred for approximately thirty minutes until a clear solution was formed;
- Sodium carboxymethylcellulose or Hydroxypropylmethylcellulose was added and stirred for another thirty minutes;
- Sodium phosphate was added and stirred;
- Finally, the pH of the final solution was adjusted to approximately seven with NaOH/HCl.

The release of Fosaprepitant Dimeglumine from the controlled release ready to use injectable solutions of example 1, was evaluated in vitro using dialysis bag membranes in a dissolution apparatus II (rotating paddles). The sustained release injectable formulations were physically separated from the bulk media by a dialysis membrane and the release was assessed from the outer bulk over time. Analytically, two to five mL of the formulation samples were placed in a dialysis bag that was sealed and placed in a vessel containing 1000 mL phosphate-buffered saline (pH 7.4, containing 0.05% sodium azide to avoid microbial growth and fungi). The test was performed at 37 ± 0.5°C under stirring at 35 rpm. The apparatus was protected from light. The results are summarized in Table 4.

**Table 4: In vitro dissolution results of tested Fosaprepitant Dimeglumine controlled release ready to use injectable solutions according to example 1.**

| Time points (h) | Reference | Formulation A \| | Formulation B |
|---|---|---|---|
| | Mean % Release | | |
| 0.5 | 16.94 | 2.38 | 1.23 |
| 1 | 21.68 | 3.02 | 1.96 |
| 4 | 37.50 | 5.75 | 5.47 |
| 12 | 49.91 | 14.72 | 14.85 |
| 24 | 63.18 | 28.42 | 29.29 |
| 36 | 71.61 | 41.39 | 41.12 |
| 48 | 80.90 | 52.04 | 53.62 |
| 72 | 91.30 | 73.32 | 75.63 |

Results of the in vitro dissolution study indicated that both tested formulations (A and B) showed a controlled release profile. Analytically, the reference formulation exhibited increased initial burst release, while the 63.18 % of the active substance was released in day one (24 h).

The two tested formulations reduced the initial burst effect of the first hour compared to the reference formulation. Additionally, both formulations had the required zero order release profile.

The stability of Fosaprepitant Dimeglumine in the prepared controlled release ready to use injectable solutions was evaluated for up to 12 days at 5°C and 25°C. Results are summarized in Table 5.

**Table 5: Stability study for the tested controlled release ready to use injectable solutions.**

| Storage conditions | | Assay (% Fosaprepitant Dimeglumine) | | |
|---|---|---|---|---|
| Time (days) | Temperature | Reference | Formulation A | Formulation B |
| 0 | 5 | 100.00 | 100.00 | 100.00 |
| | 25 | 100.04 | 100.00 | 100.98 |
| 3 | 5 | 100.00 | 100.00 | 100.00 |
| | 25 | 98.67 | 100.01 | 97.44 |
| 4 | 5 | 100.00 | 100.82 | 100.08 |
| | 25 | 97.20 | 100.00 | 92.95 |
| 5 | 5 | 100.00 | 100.00 | 98.63 |
| | 25 | 96.39 | 99.95 | 92.56 |
| 6 | 5 | 99.35 | 100.00 | 97.09 |
| | 25 | 91.58 | 98.12 | 90.62 |
| 7 | 5 | 98.34 | 100.00 | 95.75 |
| | 25 | 93.00 | 87.83 | 88.28 |
| 12 | 5 | 96.62 | 98.88 | 90.11 |
| | 25 | 86.59 | 77.81 | 82.31 |

The reference formulation was stable under storage at 25°C for up to 4 days and up to 7 days at 5°C. Formulation A (with HPMC K100 as viscosity increasing agent) exhibited good storage stability at 5°C for up to 12 days, while formulation B (with SCMC) was stable at the same conditions for up to 6 days.

Both tested controlled release ready to use solutions were able to achieve optimum dissolution goals (reduced initial burst effect and zero order release profile for at least 3 days) but only formulation A (with HMPC) was able to retain Fosaprepitant Dimeglumine's hydrolysis at typical storage conditions.

### Example 2

Non-aqueous ready to use suspensions according to the present invention are illustrated in Table 6 below.

**Table 6: Controlled release non-aqueous ready to use injectable suspensions**

| | Formulation 1 | Formulation 2 | Formulation 3 | Formulation 4 |
|---|---|---|---|---|
| Fosaprepitant Dimeglumine | 142.7 mg.mL⁻¹ | 142.7 mg.mL⁻¹ | 142.7 mg.mL⁻¹ | 142.7 mg.mL⁻¹ |
| Castor oil | 2 mL | - | - | - |
| Sesame oil | - | 2 mL | - | - |
| Cotton seed | - | - | 2 mL | - |
| Ethyl-oleate | - | - | - | 2 mL |

Sodium Phosphate and NaOH or HCl were used at appropriate amounts in order to adjust pH at 7.

A variety of fixed oils were tested as appropriate non-aqueous vehicles. Specifically, four formulations were prepared using: 1) castor oil, 2) sesame oil, 3) cotton seed oil and 4) ethyl-oleate, as non-aqueous vehicles. Micronized Fosaprepitant Dimeglumine was used at a concentration of 142.7 mg·ml⁻¹ in all formulations.

The controlled release ready to use injectable suspensions of Fosaprepitant Dimeglumine of example 2 were prepared by the following process:
- Weighted amounts of Fosaprepitant Dimeglumine and non-aqueous vehicle were mixed into a glass vial, or a beaker and stirred for approximately thirty minutes;
- Sodium Phosphate was added while stirring until a good suspension was observed;
- The pH of the final suspension was adjusted to approximately seven with NaOH/HCl.

The in vitro release of Fosaprepitant Dimeglumine from the controlled release ready to use injectable suspensions was evaluated using the same apparatus and experimental conditions described in the case of injectable solutions (example 1). The optimum formulation should exhibit a zero order release profile for at least 3 days (72 h), with low initial burst release.

The stability of Fosaprepitant Dimeglumine in the prepared controlled release ready to use injectable suspensions was evaluated for up to 12 days at 5°C and 25°C. The concentration of Fosaprepitant Dimeglumine and Aprepitant was determined by HPLC method.

Eighteen gauge needles were used to fill 1 ml of the samples in the dialysis bag membranes for the in vitro dissolution study. The results are summarized in Table 7.

**Table 7: In vitro dissolution results of tested Fosaprepitant Dimeglumine controlled release non-aqueous ready to use injectable suspensions with various fixed oils**

| | Formulation 1 | Formulation 2 | Formulation 3 | Formulation 4 |
|---|---|---|---|---|
| Time points (h) | %Mean Release | | | |
| 1 | 2.77 | 0.69 | 2.89 | 2.82 |
| 8 | 7.23 | 4.46 | 7.34 | 7.28 |
| 12 | 12.87 | 21.16 | 29.52 | 11.78 |
| 72 | 78.08 | >80 | >65 | 61.45 |

The *in vitro* dissolution study indicated that all tested non-aqueous suspensions exhibited sustained release profiles with limited initial burst release. Contrary to castor oil (formulation 1) and ethyl-oleate (formulation 4) formulations, were a constant release rate was observed for at least 72 hours, sesame oil (formulation 2) and cotton seed oil (formulation 3) formulations exhibited a burst release at 12 hours. Both castor oil and ethyl-oleate formulations showed a zero order release profile with R² = 0.986 for castor oil, and R² = 0.985 for ethyl-oleate. Hence, those two formulations were able to fulfill the optimum dissolution goals as both reduced the initial burst effect of the tested active ingredient and exhibited a zero order release profile for at least 3 days.

In a further step, the effect of the Fosaprepitant Dimeglumine concentration on the *in vitro* dissolution profile was investigated. Hence, a new castor oil formulation was prepared with active ingredient concentration of 57.5 mg·ml⁻¹. Sodium Phosphate and NaOH or HCl were used at appropriate amounts in order to adjust pH at 7.

Eighteen gauge needles were used to fill 1 mL of the sample in the dialysis bag membranes for the *in vitro* dissolution study. The results are summarized in Table 8.

**Table 8: In vitro dissolution results of castor oil controlled release injectable suspension with 57.5 mg·ml⁻¹ Fosaprepitant Dimeglumine.**

| Time points (h) | Mean % Release |
|---|---|
| 1 | 1.76 |
| 8 | 10.96 |
| 12 | 15.20 |
| 72 | 76.51 |

Comparison of the *in vitro* dissolution profiles with the aid of similarity factor (f₂) showed that the concentration of the active ingredient in the tested formulation had an insignificant effect (f₂ = 78.77), while both formulations (with 57.5 and 142.7 mg·ml⁻¹ Fosaprepitant Dimeglumine) showed a constant zero order release rate for at least 3 days with limited initial burst release.

The stability study of all tested non-aqueous suspensions indicated that the active ingredient was stable under all tested storage conditions (25°C and 5°C).

### Example 3

The composition of a sol-gel injectable formulation according to the present invention requires the presence of a biodegradable polymer. Various polymers were tested with respect to the phase transition according to their concentration and temperature. Poloxamer 108, poloxamer 408, PLLA-PEG 1100:600 copolymer, PLLA-PEG 1300:600 copolymer, PLLA-PEG 5000:2000 copolymer, PLLA-PEG 5000:3000 copolymer, PLLA-PEG 5000:5000 copolymer and mixture of poloxamer/PLLA-PEG 1100:600 copolymer were some of the tested biodegradable polymers.

The sol (flow) - gel (no flow) transition temperature was determined (with precision of ±0.5°C) by the test tube inverting method with temperature increments of 2°C per each step. Each sample with a given concentration was prepared by dissolving the polymer in either distilled water or phosphate-buffered saline (pH 7.4) in a 4 mL vial. After equilibration at 20°C for 20 min, the vials containing the samples were immersed in a water bath at a constant designed temperature for 20 min. inverting the vial determined a gel state when no fluidity in 1 min was visually observed.

The phase transition (sol-gel) of the biodegradable polymers tested in the current invention was studied by the test tube inversion method. Initially, the sol-gel transition of aqueous poloxamer (nonionic polyoxyethylene-polyoxypropylene copolymer) solutions was tested in water and phosphate-buffered saline. Two types of poloxamers were tested: 1) type 188 (at concentrations of 7.5%w/v, 10%w/v, 15%w/v and 20%w/v) and 2) type 407 (at concentrations of 14%w/v, 16%w/v, 18%w/v and 20%w/v). The type number specifies the approximate average molecular weight of the polyoxypropylene portion and the percentage by weight of the polyoxyethylene portion.

None of the tested poloxamer 188 solutions was able to form a gel in the region of the examined temperature (20-60°C). Poloxamer 407 solutions at concentrations below 16% (w/v) did not undergo sol-gel transition as well. However, at 16% (w/v) concentration a sol-gel transition of the poloxamer 407 solution is observed at 37°C, while as the temperature rises (over 38°C) the gel phase turns into a turbid solution again. Sol-gel transition is observed for all solution with concentration greater than 16 % (w/v) at 28°C, while no reversible gel-sol transition due to the rise of temperature was observed at least for up to 60°C.

In a further step, the phase transition of aqueous PLLA-PEG copolymer solutions was tested in water and phosphate-buffered saline. Five types of PLLA-PEG copolymers namely: 1) 1100:600,2) 1300:600, 3) 5000:2000, 4) 5000:3000, and 5) 5000:5000; at concentrations from 7.5%w/v to 20%w/v were tested. The two numbers indicate the molecular weight (in Da) of the PLLA and PEG, respectively. The test tube inverting method was also used.

At concentrations below 10% (w/v) PLLA-PEG 1100:600 did not undergo sol-gel transition. For 10% (w/v) concentration a sol-gel transition at 48°C was observed in water, while in phosphate-buffered saline had no phase transition. All samples with concentration below 18 % (w/v) the sol-gel transition temperature is lower when the polymer is tested in a phosphate-buffered saline medium, compared to distilled water. Analytically, at concentration of 12 % (w/v) the sol-gel transition temperature is 52°C in water and 40°C in phosphate-buffered saline, at 14 % (w/v) is 40°C in water and 34°C in phosphate-buffered saline, at 16 % (w/v) is 34°C in water and 26°C in phosphate-buffered saline, at 18 % (w/v) is 34°C in water and 26°C in phosphate-buffered saline, and at 20 % (w/v) in both water and in phosphate-buffered saline is at 20°C. No reversible phase transition (gel to sol) with increasing temperature was observed.

At concentrations below 14 % (w/v) PLLA-PEG 1300:600 did not undergo sol-gel transition, while a double revisable sol-gel transition is observed for both water and phosphate-buffered saline at a concentration of 14 % (w/v). Specifically, at 40°C a sol to gel transition undergoes in both water and phosphate-buffered saline, while a reversible gel to sol transition is observed at 56°C for water and 54°C for phosphate-buffered saline. A double reversible transition from sol to gel, for the 14 % (w/v) PLLA-PEG 1300:600, is observed at 58°C in both cases. At a concentration of 16 % (w/v) a sol-gel transition is observed at 28°C (in water and phosphate-buffered saline), while at a higher concentration the sol-gel transition undergoes at temperatures equal or below 24°C.

Finally, the phase transition experiments (from 20°C to 60°C) for PLLA-PEG 5000:2000, 5000:3000 and 5000:5000 copolymer indicated that there was no sol-gel transition for concentrations varying from 7.5 % (w/v) to 20 % (w/v).

In a further step, the phase transition of mixtures consisting both poloxamer 407 and PLLA-PEG 1100:600 copolymers in phosphate-buffered saline and at varying concentration levels was examined. All polymer mixtures did not undergo sol-gel phase transition.

It is important to note that the accurate determination of the sol-gel transition temperature is of primary importance when designing an in situ gel forming system, as the mixture of the active ingredient and polymer solution (or suspension with good flowability and injectability) is transformed into a hydrogel depot when injected (at 37°C). Hence, from the phase transition experiments conducted in the present invention it is concluded that poloxamer 407, PLLA-PEG 1100:600 and PLLA-PEG 1300:600 were able to undergo a desirable sol-gel transition (solution at room temperature and gel at 37°C), for the preparation of a sustained release injectable Aprepitant or Fosaprepitant formulation.

The composition of a sol-gel injectable formulation according to the present invention is illustrated in Table 9:

**Table 9: Composition of controlled release injectable sol-gel formulation containing PLLA-PEG 1100:600 or poloxamer 407.**

| | Formulation 1 | Formulation 2 |
|---|---|---|
| Fosaprepitant Dimeglumine | 142.6 mg.mL⁻¹ | 142.6 mg.mL⁻¹ |
| PLLA-PEG 1100-600 | 16% (w/v) | - |
| Poloxamer 407 | - | 16% (w/v) |
| Water for injection | q.s 1 to 3 mL | q.s 1 to 3 mL |

Sodium Phosphate and NaOH or HCl were used at appropriate amounts in order to adjust pH at 7.

The controlled release sol-gel injectable formulation of Fosaprepitant Dimeglumine was prepared by the following process:
- Weighted amounts of Fosaprepitant Dimeglumine and water were mixed into a glass vial, or a beaker, stir for approximately thirty minutes until a clear solution is made;
- Weighted amount of PLLA-PEG 1100-600 or poloxamer 407 was added and stirred until a good suspension was observed;
- Sodium Phosphate was added and stirred;
- Finally, the pH of the final formulation was adjusted to approximately seven with NaOH/HCl.

The *in vitro* dissolution profile of the two formulations above, containing poloxamer 407 and PLLA-PEG 1100:600 as sol-gel biodegradable polymers was determined. The in vitro release of Fosaprepitant Dimeglumine from the sol-gel injectable formulation was evaluated using the same apparatus and experimental conditions as in the case of injectable solutions. The optimum formulation should exhibit a zero order release profile for at least 3 days (72 h), with low initial burst release. The prepared formulations showed good flowability and syringability-injectability at room temperature with no clogging or blockage of the syringe needles.

Eighteen (18) gauge needles were used to fill 2mL of the samples in the dialysis bag membranes for the dissolution study. The results are summarized in Table 10 and the release profiles are depicted in Fig.5.

**Table 10: In vitro dissolution results of preliminary controlled release injectable sol-gel formulations containing poloxamer 407 (16% w/v) and PLLA-PEG 1100:600 (16% w/v).**

| Tested formulation | Time points (h) | Mean % Release |
|---|---|---|
| Poloxamer 407 | 1 | 21.43 |
| | 8 | 55.77 |
| | 24 | 75.97 |
| | 72 | 92.40 |
| PLLA-PEG 1100-600 | 1 | 3.71 |
| | 8 | 9.24 |
| | 24 | 24.68 |
| | 72 | 60.72 |

The *in vitro* dissolution study indicated that the sol-gel formulation with poloxamer 407 exhibited an increased initial burst release, with 21.43 % of Fosaprepitant Dimeglumine being released at the first hour, while the formulation was also unable to successfully sustain the active ingredient's release (75.97 % of the active ingredient was released in day 1). On the other hand, the PLLA-PEG sol-gel formulation showed a zero order release profile (R² = 0.993) with no burst effect (3.71 % of the active ingredient was released in the first hour), while 60.72 % of Fosaprepitant Dimeglumine was released in 3 days.

Hence, although both poloxamer 407 and PLLA-PEG were able to create an acceptable sol-gel vehicle (sol-gel transition temperature close to 37°C), only the latter was able to produce a desirable dissolution profile for Fosaprepitant Dimeglumine (zero order release for at least 3 days, with low initial burst release).

In a further step, the *in vitro* dissolution profile of several PLLA-PEG 1100:600 sol-gel formulations at varying concentrations (8%w/v, 10%w/v and 12%w/v) was evaluated. The prepared formulations showed good syringability and injectability with no clogging or blockage of the syringe needles. Sodium Phosphate and NaOH or HCl was used at appropriate amounts in order to adjust pH at 7.

Eighteen gauge needles were used to fill 2 mL of the samples in the dialysis bag membranes for the *in vitro* dissolution study. The results are summarized in Table 11.

**Table 11: In vitro dissolution results for PLLA-PEG 1100:600 sol-gel formulations with varying copolymer concentrations.**

| Concentration of PLLA-PEG | Time points (h) | Mean % Release |
|---|---|---|
| 8% (w/v) | 1 | 0 |
| | 6 | 12.43 |
| | 12 | 23.77 |
| | 24 | 41.45 |
| | 72 | 90.75 |
| 10% (w/v) | 1 | 2.43 |
| | 6 | 8.11 |
| | 12 | 22.96 |
| | 24 | 34.87 |
| | 72 | 73.90 |
| 12% (w/v) | 1 | 0 |
| | 6 | 4.57 |
| | 12 | 14.10 |
| | 24 | 32.09 |
| | 72 | 90.98 |

The *in vitro* dissolution study indicated that the formulation with 10 % (w/v) PLLA-PEG 1100:600 exhibited an increased initial burst release at 12 hours, while the rest formulations showed limited burst release and a close to zero order release profile, with R² = 0.972, 0.995 and 0.966 for 8%, 10% and 12% (w/v) PLLA-PEG, respectively.

Additionally, another formulation, containing 8 % (w/v) PLLA-PEG and 1 wt% SCMC (based on the total weight of the formulations) as viscosity increasing agent was also tested. Analytically, formulation with 8% PLLA-PEG and 1 wt% SCMC released 12.43 % and 90.75% of Fosaprepitant Dimeglumine in 6 and 72 hours respectively.

It is concluded that formulation with 12% (w/v) PLLA-PEG was able to fulfill the optimum dissolution goals with low initial burst release and zero order release profile for at least 3 days. Similarity factor's value of 74.96 for the optimum dissolution profile and the 12% (w/v) PLLA-PEG formulation indicated increased similarity. Finally, the stability study indicated that Fosaprepitant Dimeglumine was stable in all formulations under the selected storage conditions.

## Claims

1. A controlled release pharmaceutical composition for intramuscular or subcutaneous administration comprising Aprepitant or Fosaprepitant or pharmaceutical acceptable salt, derivative or metabolite thereof, as the active ingredient and a viscosity increasing agent at a concentration varying from 0.01 mg/ml to 350 mg/ml based on the total weight of the formulation, for the treatment of Chemotherapy Induced Nausea and Vomiting Syndrome, wherein the active substance is released in a constant rate for at least three days after administration.

2. The pharmaceutical composition according to claim 1, that is in the form of an aqueous ready to use solution or non-aqueous ready to use suspension or composition that is in solution but forms a gel after administration.

3. The pharmaceutical composition according to claim 1, wherein the viscosity increasing agent is at a concentration from 1 mg/ml to 150 mg/ml based on the total weight of the formulation.

4. The pharmaceutical composition according to claim 3, wherein the viscosity enhancing agent is one selected from aluminum monostearate, carboxy methylcellulose, desoxycholate sodium, gelatin, glycerol, hydroxyethyl cellulose, hydroxypropylmethylcellulose, lecithin, polyoxyethylene alkyl ethers, poloxamer, polyoxyethylated fatty acid, polysorbate, polyethylene glycol, polyvinyl pyrrolidone and sodium carboxymethylcellulose.

5. The pharmaceutical composition according to claim 1, wherein it further comprises a non-aqueous water-immiscible liquid vehicle such as sesame or castor or ethyl-oleate or cotton seed oil or mixtures thereof.

6. The pharmaceutical composition according to claim 1, wherein it further comprises a biodegradable polymer that has a molecular weight from 1,000 Da up to over 100,000 Da.

7. The pharmaceutical composition according to claim 6, wherein the biodegradable polymer is one of Poloxamer 108, poloxamer 408, PLLA-PEG 1100:600 copolymer, PLLA-PEG 1300:600 copolymer, PLLA-PEG 5000:2000 copolymer, PLLA-PEG 5000:3000 copolymer, PLLA-PEG 5000:5000 copolymer and mixture of poloxamer/PLLA-PEG 1100:600.

8. The pharmaceutical composition according to any preceding claim, wherein it further comprises one or more excipients selected from solubilizing/wetting, buffering, pH adjusting, antioxidants, preservatives, suspending/flocculating or chelating agents.

9. A process for preparing a controlled release injectable formulation for intramuscular or subcutaneous administration comprising Aprepitant or Fosaprepitant or pharmaceutical acceptable salt, derivative or metabolite thereof, as the active ingredient for the treatment of Chemotherapy Induced Nausea and Vomiting Syndrome, wherein the active substance is released in a constant rate for at least three days after administration, which process comprises the following steps:
- Weighted amounts of the active ingredient and liquid vehicle (aqueous or non-aqueous) are mixed into a glass vial, or a beaker and stirred for approximately thirty minutes until a clear solution is achieved
- A viscosity increasing agent at a concentration varying from 0.01 mg/ml to 350 mg/ml based on the total weight of the formulation and any optional pharmaceutically acceptable excipient such as a biodegradable polymer is added and stirred for another thirty minutes.
- Finally, the pH of the final solution is adjusted to approximately seven.

10. A process for preparing a controlled release injectable formulation for intramuscular or subcutaneous administration comprising Aprepitant or Fosaprepitant or pharmaceutical acceptable salt, derivative or metabolite thereof, as the active ingredient for the treatment of Chemotherapy Induced Nausea and Vomiting Syndrome, wherein the active substance is released in a constant rate for at least three days after administration, which process comprises the following steps:
- A viscosity increasing agent at a concentration varying from 0.01 mg/ml to 350 mg/ml based on the total weight of the formulation and weighted amounts of any optional pharmaceutically acceptable excipient such as a biodegradable polymer and liquid vehicle (aqueous or non-aqueous) are mixed into a glass vial, or a beaker and stirred for approximately thirty minutes until a clear solution is achieved
- The pharmaceutically active ingredient is added and stirred for another thirty minutes.
- Finally, the pH of the final solution is adjusted to approximately seven.

11. A process for preparing a controlled release injectable formulation for intramuscular or subcutaneous administration comprising Aprepitant or Fosaprepitant or pharmaceutical acceptable salt, derivative or metabolite thereof, as the active ingredient for the treatment of Chemotherapy Induced Nausea and Vomiting Syndrome, wherein the active substance is released in a constant rate for at least three days after administration, which process comprises the following steps:
- Two distinct solutions are prepared. A first solution is prepared by mixing a viscosity increasing agent at a concentration varying from 0.01 mg/ml to 350 mg/ml based on the total weight of the formulation weighted amounts and any optional pharmaceutically acceptable excipient such as a biodegradable polymer, with a liquid vehicle (aqueous or non-aqueous) into a glass vial or a beaker and stirred for approximately thirty minutes
- A second solution is prepared by mixing weighted amounts of the active ingredient and liquid vehicle (aqueous or non-aqueous) into a glass vial, or a beaker and stirring for approximately thirty minutes.
- Finally, the two solutions are then mixed together at appropriate amounts. The pH of the final solution is adjusted to approximately seven.

## Patentansprüche

1. Pharmazeutische Zusammensetzung mit kontrollierter Freisetzung zur intramuskulären oder subkutanen Verabreichung, umfassend Aprepitant oder Fosaprepitant oder ein pharmazeutisch verträgliches Salz, Derivat oder Metabolit davon als Wirkstoff und ein viskositätssteigerndes Mittel in einer Konzentration, die von 0,01 mg/ml bis 350 mg/ml variiert, bezogen auf Gesamtgewicht der Formulierung zur Behandlung von durch Chemotherapie hervorgerufener Übelkeit und Erbrechen, wobei der Wirkstoff für mindestens drei Tage nach der Verabreichung mit einer konstanten Geschwindigkeit freigesetzt wird.

2. Pharmazeutische Zusammensetzung nach Anspruch 1, die in Form einer wässrigen gebrauchsfertigen Lösung oder einer nicht-wässrigen gebrauchsfertigen Suspension oder Zusammensetzung vorliegt, die in Lösung vorliegt, aber nach der Verabreichung ein Gel bildet.

3. Pharmazeutische Zusammensetzung nach Anspruch 1, wobei das viskositätserhöhende Mittel eine Konzentration von 1 mg/ml bis 150 mg/ml aufweist, bezogen auf das Gesamtgewicht der Formulierung.

4. Pharmazeutische Zusammensetzung nach Anspruch 3, wobei das viskositätserhöhende Mittel eines ist, das ausgewählt ist aus Aluminiummonostearat, Carboxymethylcellulose, Desoxycholat-Natrium, Gelatine, Glycerin, Hydroxyethylcellulose, Hydroxypropylmethylcellulose, Lecithin, Polyoxyethylenalkylethern, Poloxamer, polyoxyethylierter Fettsäure, Polysorbat, Polyethylen Glykol, Polyvinylpyrrolidon und Natriumcarboxymethylcellulose.

5. Pharmazeutische Zusammensetzung nach Anspruch 1, **dadurch gekennzeichnet, dass** sie weiterhin ein nichtwässriges mit Wasser nicht mischbares flüssiges Vehikel wie Sesam oder Rizinus oder Ethyloleat oder Baumwollsamenöl oder Gemische davon enthält.

6. Pharmazeutische Zusammensetzung nach Anspruch 1, wobei sie ferner ein biologisch abbaubares Polymer umfasst, das ein Molekulargewicht von 1.000 Da bis über 100.000 Da aufweist.

7. Pharmazeutische Zusammensetzung nach Anspruch 6, wobei das biologisch abbaubare Polymer eines von Poloxamer 108, Poloxamer 408, PLLA-PEG 1100: 600-Copolymer, PLLA-PEG 1300: 600-Copolymer, PLLA-PEG 5000: 2000-Copolymer, PLLA-PEG 5000:3000 Copolymer, PLLA-PEG 5000: 5000 Copolymer und Mischung aus Poloxamer / PLLA-PEG 1100: 600 ist.

8. Pharmazeutische Zusammensetzung nach einem der vorhergehenden Ansprüche, **dadurch gekennzeichnet, dass** sie weiterhin einen oder mehrere Hilfsstoffe enthält, die ausgewählt sind aus Lösungsvermittlern/Benetzern, Puffern, Einstellen des pH-Werts, Antioxidationsmitteln, Konservierungsmitteln, Suspendiermitteln/Flockungsmitteln oder Chelatbildnern.

9. Verfahren zur Herstellung einer injizierbaren Formulierung mit kontrollierter Freisetzung zur intramuskulären oder subkutanen Verabreichung, umfassend Aprepitant oder Fosaprepitant oder ein pharmazeutisch verträgliches Salz, Derivat oder Metabolit davon als Wirkstoff zur Behandlung von durch Chemotherapie induzierter Übelkeit und Erbrechen, wobei der Wirkstoff freigesetzt wird in eine konstante Rate für mindestens drei Tage nach der Verabreichung, wobei das Verfahren die folgenden Schritte umfasst:
- Gewichtete Mengen des Wirkstoffs und des flüssigen Trägers (wässrig oder nicht wässrig) werden in ein Glasfläschchen oder ein Becherglas gemischt und ungefähr 30 Minuten gerührt, bis eine klare Lösung erreicht ist
- Ein viskositätserhöhendes Mittel in einer Konzentration von 0,01 mg/ml bis 350 mg/ml, bezogen auf das Gesamtgewicht der Formulierung, und ein beliebiger pharmazeutisch verträglicher Hilfsstoff wie ein biologisch abbaubares Polymer werden zugegeben und weitere 30 Minuten gerührt.
- Schließlich wird der pH-Wert der endgültigen Lösung auf ungefähr sieben eingestellt.

10. Verfahren zur Herstellung einer injizierbaren Formulierung mit kontrollierter Freisetzung zur intramuskulären oder subkutanen Verabreichung, umfassend Aprepitant oder Fosaprepitant oder ein pharmazeutisch verträgliches Salz, Derivat oder Metabolit davon als Wirkstoff zur Behandlung von durch Chemotherapie induzierter Übelkeit und Erbrechen, wobei der Wirkstoff freigesetzt wird in eine konstante Rate für mindestens drei Tage nach der Verabreichung, wobei das Verfahren die folgenden Schritte umfasst:
- ein viskositätserhöhendes Mittel in einer Konzentration, die von 0,01 mg/ml bis 350 mg/ml variiert, bezogen auf das Gesamtgewicht der Formulierung und gewichtete Mengen eines beliebigen pharmazeutisch verträglichen Hilfsstoffs, wie eines biologisch abbaubaren Polymers und eines flüssigen Trägers (wässrig oder nicht wässrig) werden in ein Glasfläschchen oder ein Becherglas gemischt und etwa 30 Minuten gerührt, bis eine klare Lösung erreicht ist
- Der pharmazeutische Wirkstoff wird zugegeben und weitere 30 Minuten gerührt.
- Schließlich wird der pH-Wert der endgültigen Lösung auf ungefähr sieben eingestellt.

11. Verfahren zur Herstellung einer injizierbaren Formulierung mit kontrollierter Freisetzung zur intramuskulären oder subkutanen Verabreichung, umfassend Aprepitant oder Fosaprepitant oder ein pharmazeutisch verträgliches Salz, Derivat oder Metabolit davon als Wirkstoff zur Behandlung von durch Chemotherapie induzierter Übelkeit und Erbrechen, wobei der Wirkstoff freigesetzt wird in eine konstante Rate für mindestens drei Tage nach der Verabreichung, wobei das Verfahren die folgenden Schritte umfasst:
- Es werden zwei verschiedene Lösungen hergestellt. Eine erste Lösung wird hergestellt, indem ein viskositätserhöhendes Mittel in einer Konzentration, die von 0,01 mg/ml bis 350 mg/ml variiert, basierend auf dem Gesamtgewicht der formulierungsgewichteten Mengen und einem beliebigen pharmazeutisch verträglichen Hilfsstoff, wie einem biologisch abbaubaren Polymer, mit einer Flüssigkeit gemischt wird Träger (wässrig oder nicht wässrig) in ein Glasfläschchen oder ein Becherglas und etwa 30 Minuten lang gerührt
- Eine zweite Lösung wird hergestellt, indem gewichtete Mengen des Wirkstoffs und des flüssigen Trägers (wässrig oder nicht wässrig) in ein Glasfläschchen oder ein Becherglas gemischt und etwa 30 Minuten gerührt werden.
- Schließlich werden die beiden Lösungen in geeigneten Mengen miteinander vermischt. Der pH-Wert der endgültigen Lösung wird auf ungefähr sieben eingestellt.

## Revendications

1. Une composition pharmaceutique à libération contrôlée pour administration intramusculaire ou sous cutanée comprenant de l'Aprépitant ou du Fosaprépitant ou un sel pharmaceutiquement acceptable, un dérivé ou un métabolite de celui-ci, comme principe actif et un agent augmentant la viscosité à une concentration variant de 0.01 mg/ml à 350 mg/ml basée sur le poids total de la formulation, pour le traitement du Syndrome de Nausées et Vomissements Induits par la Chimiothérapie, dans laquelle le principe actif est libéré à un taux constant pendant au moins trois jours après administration.

2. La composition pharmaceutique selon la revendication 1, qui se présente sous la forme d'une solution aqueuse prête à l'emploi ou d'une suspension non-aqueuse prête à l'emploi ou d'une composition qui est en solution mais qui forme un gel après administration.

3. La composition pharmaceutique selon la revendication 1, dans laquelle l'agent augmentant la viscosité est présent à une concentration de 1 mg/ml à 150 mg/ml basée sur le poids total de la formulation.

4. La composition pharmaceutique selon la revendication 3 dans laquelle l'agent augmentant la viscosité est l'un choisi parmi le monostéarate d'aluminium, le carboxy méthylcellulose, le désoxycholate de sodium, la gélatine, le glycérol, l'hydroxyéthylcellulose, l'hydroxypropylméthylcellulose, la lécithine, les éthers alkyliques de polyoxyéthylène, le poloxamère, l'acide gras polyoxyéthylé, le polysorbate, le polyéthylène glycol, la polyvinyl pyrrolidone et la carboxyméthylcellulose de sodium.

5. La composition pharmaceutique selon la revendication 1, dans laquelle est compris en outre un véhicule liquide non-aqueux et non-miscible à l'eau tel que l'huile de graines de sésame, ou de ricin, ou d'oléate d'éthyle ou de coton ou mélanges de celles-ci.

6. La composition pharmaceutique selon la revendication 1, dans laquelle est compris en outre un polymère biodégradable qui a un poids moléculaire de 1,000 Da à plus de 100,000 Da.

7. La composition pharmaceutique selon la revendication 6, dans laquelle le polymère biodégradable est l'un parmi le poloxamère 108, le poloxamère 408, PLLA-PEG 1100:600 copolymère, PLLA-PEG 1300:600 copolymère, PLLA-PEG 5000:2000 copolymère, PLLA-PEG 5000:5000 copolymère et mélange de poloxamère/PLLA-PEG 1100:600.

8. La composition pharmaceutique selon l'une quelconque des revendications précédentes, dans laquelle est compris en outre un ou plusieurs excipients sélectionnés parmi des agents solubilisant, des agents mouillant, des agents tampons, des agents d'ajustement du pH, des antioxydants, des préservatifs, des agents de suspension, des agents floculant ou des agents chélateurs.

9. Un procédé pour préparer une formulation injectable à libération contrôlée pour administration intramusculaire ou sous cutanée comprenant l'Aprépitant ou le Fosaprépitant ou sel pharmaceutiquement acceptable, dérivative ou métabolite de celui-ci, comme principe actif pour le traitement du Syndrome de Nausées et Vomissements Induits par la Chimiothérapie, dans laquelle le principe actif est libéré à un taux constant pendant au moins trois jours après administration, le dit procédé comprenant les étapes suivantes:
- les quantités pesées de principe actif et de véhicule liquide (aqueux ou non-aqueux) sont mélangées dans un flacon en verre ou un bécher et mélangées pendant approximativement trente minutes jusqu'à l'obtention d'une solution limpide;
- un agent augmentant la viscosité à une concentration variant de 0.01 mg/ml à 350 mg/ml basée sur le poids total de la formulation et tout excipient pharmaceutiquement acceptable facultatif tel qu'un polymère biodégradable sont ajoutés et mélangés pour encore trente minutes;
- finalement, le pH de la solution finale est ajusté à approximativement sept.

10. Un procédé pour préparer une formulation injectable à libération contrôlée pour administration intramusculaire ou sous cutanée comprenant l'Aprépitant ou le Fosaprépitant ou un sel pharmaceutiquement acceptable, un dérivative ou métabolite de celui-ci, comme principe actif pour le traitement du Syndrome de Nausées et Vomissements Induits par la Chimiothérapie, dans laquelle le principe actif est libéré à un taux constant pendant au moins trois jours après administration, le dit procédé comprenant les étapes suivantes:
- un agent augmentant la viscosité à une concentration variant de 0.01 mg/ml à 350 mg/ml basée sur le poids total de la formulation et des quantités pesées de tout excipient pharmaceutiquement acceptable facultatif tel qu'un polymère biodégradable et un véhicule liquide (aqueux ou non-aqueux) sont mélangés dans un flacon en verre, ou un bécher et mélangés pendant trente minutes jusqu'à l'obtention d'une solution limpide;
- le principe pharmaceutique actif est ajouté et mélangé pour encore trente minutes;
- finalement, le pH de la solution finale est ajusté à environ sept.

11. Un procédé pour préparer une formulation injectable à libération contrôlée pour administration intramusculaire ou sous-cutanée comprenant l'Aprépitant ou le Fosaprépitant ou un sel pharmaceutiquement acceptable, un dérivative ou métabolite de celui-ci, comme principe actif pour le traitement du Syndrome de Nausées et Vomissements Induits par la Chimiothérapie, dans laquelle le principe actif est libéré à un taux constant pendant au moins trois jours après administration, le dit procédé comprenant les étapes suivantes:
- deux solutions distinctes sont préparées. Une première solution est préparée en mélangeant un agent augmentant la viscosité à une concentration variant de 0.01 mg/ml à 350 mg/ml basée sur le poids total de la formulation et les quantités pesées de tout excipient pharmaceutiquement acceptable facultatif tel qu'un polymère biodégradable avec un véhicule liquide (aqueux ou non-aqueux) dans un flacon en verre ou un bécher et mélangée pendant 30 minutes;
- une deuxième solution est préparée en mélangeant des quantités pesées du principe actif et du véhicule liquide (aqueux ou non-aqueux) dans un flacon en verre ou un bécher et mélangée pendant trente minutes;
- finalement, les deux solutions sont ensuite mélangées ensemble en quantités appropriées. Le pH de la solution finale est ajusté à approximativement sept.
